# EUROPEAN PATENT APPLICATION

(11) **EP 3 650 545 A1**
(43) Date of publication of application: **13.05.2020**
(21) Application number: 18820610.6
(22) Date of filing: 19.06.2018
(51) Int. Cl.: C12N 15/113, C12N 5/10

(54) **METHOD FOR KNOCKING OUT TARGET GENE IN T CELLIN VITRO**

(30) Priority: 20.06.2017 CN 201710469539
(71) Applicant: Jiangsu Hengrui Medicine Co., Ltd., Jiangsu 222047 (CN); Shanghai Hengrui Pharmaceutical Co., Ltd., Shanghai 200245 (CN)
(72) Inventor: PENG, Zuohan, Shanghai 200245 (CN); SHEN, Lianjun, Shanghai 200245 (CN); TAO, Weikang, Shanghai 200245 (CN)
(74) Representative: Gevers & Orès
(86) International application number: PCT/CN2018/091804
(87) International publication number: WO 2018/233596

(57) **Abstract**

Provided is a method for knocking out target genes in T cells *in vitro* based on the CRISPR-Cas9 system. Also provided are crRNA targeting target genes TRAC, B2M and PD1, and a kit comprising sgRNA formed by linking the crRNA and tracrRNA corresponding to a Cas9 protein, the Cas9 protein, an oligodeoxyribonucleic acid (N-oligo) or a milt DNA fragment. The kit is used to knock out the TCR, B2M and/or PD1 genes in T cells. Also provided are T cells with gene knock out obtained according to the method of the present invention and the use thereof.

## Description

### FIELD OF THE INVENTION

The invention belongs to the field of biological medicine. Specifically, it relates to a method for knocking out target gene in T cells *in vitro,* a crRNA used in the method, the T cells obtained by the method and use thereof.

### BACKGROUND OF THE INVENTION

Adoptive cell therapy (ACT), involving the transfer of autologous antigen-specific T cells produced *ex vivo,* is a promising strategy for the treatment of viral infections and cancers. T cells used in adoptive cell therapy may be generated by amplification of antigen-specific T cells or by redirection of genetically designed T cells (Park, Rosenberg et al. Trends Biotechnol. 2011, 29(11): 550-557).

CART is obtained by genetically inserting a chimeric antigen receptor (CAR) into isolated T cell to enhance the targeting, killing activity and persistence of the T cell, and the recognition of tumor cell surface antigens is independent of the MHC restriction. CARs consist of an extracellular antigen binding region, a transmembrane region, and an intracellular signal transduction region of T cell receptor (such as CD3ζ and costimulatory molecules). The extracellular antigen binding region is composed of a light chain variable region (VL) linked to a heavy chain variable region (VH) of monoclonal antibody via hinge, to form a single chain fragment variable (scFv), which is capable of recognizing a specific tumor antigen. Relevant clinical trials have shown that CAR has a better therapeutic effect in patients with lymphoma which is not responsive to other treatments. The study on CART-19 by Carl June of the University of Pennsylvania showed that in 45 out of 75 patients with leukemia (including adults and children) had complete remission after the treatment with CART cells.

However, the existing CART therapies typically utilize autologous lymphocytes from the tumor patients. Such lymphocytes are modified, cultured, activated, and expanded *in vitro* and then returned back to the patient. In addition to side effects such as cytokine storm, there are three major problems: firstly, CART treatment is not applicable to the advanced patients with lower number or poorer quality of lymphocytes; secondly, the efficacy of CART therapy in solid tumors is still not significant, probably due to the poorer survival rate and the lower activity of immune cells in tumor tissues resulting from the influence of the signal pathway of the immunosuppression checkpoint; finally, the cost is high and the burden on the patient is increased, because CART is an personalized treatment. Therefore, the development of universal CAR-T cells (UCART) derived from allogeneic origin will promote its application.

In the early stage, gene knock-out was performed by targeting vector via homologous recombination or by RNAi technology, but both have problems, such as cumbersome operation and low efficiency. Cellectis company has successfully cured several children patients suffered from relapsed acute lymphocyte leukemia (ALL) by directionally knocking out TCRα gene (reducing GVHD) and CD52 gene (making cells tolerant to Alemtuzumab) with allogeneic CAR-T therapy UCART19 developed by TALEN technology. However, cumbersome construction and large-scale sequencing processes are needed when knocking out TCR through TALEN proposed by Cellectis. Currently, clustered regularly interspaced short palindromic repeat (CRISPR-associated, CRISPR-Cas9) achieves gene editing by recognizing specific DNA sequence, and this is simpler and more efficient than TALEN.

Currently, there are examples of gene knockout based on CRISPR/Cas9 system, such as those mentioned in CN104395463A, CN105518146A and CN106191062A. However, there are still some problems to be solved, such as low knockout rate of the target gene, requiring several transfection or transformation processes, cumbersome operations, much damage to the T cells, or high off-target rate. Therefore, there is still a need to optimize the method for knocking out TCR gene based on CRISPR/Cas9 system and also crRNAs with higher accuracy.

### SUMMARY OF THE INVENTION

The object of the present invention is to overcome the problems present in immunotherapy in the prior art, and to provide a method for constructing TCR-negative T cells by knocking out TCR with CRISPR/Cas9 gene editing technology, and to provide TCR-negative T cells obtained by the method.

One object of the present invention is to provide TCR⁻ and PD1⁻ (or B2M⁻) double negative T cells and to provide a method for constructing the same.

Another object of the present invention is to provide TCR⁻, B2M⁻ and PD1⁻ triple negative T cells and to provide a method for constructing the same.

Further, the above TCR negative T cells, TCR⁻ and PD-1⁻ (or B2M⁻) double negative T cells and TCR/B2M/PD1 triple negative T cells are sorted by using magnetic beads, and are used in adoptive cell immunotherapy of tumors and the like.

In a first embodiment, a method for knocking out one or more target gene(s) in T cells *in vitro* is provided, comprising the steps of:
1) contacting sgRNAs targeting one or more target gene(s) in the T cells with Cas9 protein respectively, to form a protein-RNA complex (RNP);
2) mixing said RNP with an oligodeoxyribonucleic acid (N-oligo) or a milt DNA fragment, and transforming the obtained mixture into T cells, wherein the sgRNAs direct the Cas9 protein to the corresponding target sequence of the target gene and to hybridize with the target sequence, thereby the target gene is cleaved, and the cleavage efficiency of the target gene is not less than 75%.

In a preferred embodiment, in the method for knocking out one or more target gene(s) in T cells *in vitro* of the invention, wherein the target gene is one or more selected from the group consisting of TRAC, TRBC, B2M and PD1 genes, and the sgRNA targets a coding sequence of the target gene or a regulatory sequence for the expression of the target gene.

Further, in the method for knocking out one or more target gene(s) in T cells *in vitro* of the invention, wherein the sgRNA consists of, from 5' to 3': a crRNA targeting the target gene with a length of 17-20 nt which is linked to a tracrRNA corresponding to the Cas9 protein, wherein the crRNA is preferably 17 nt in length.

In a preferred embodiment, in the method for knocking out one or more target gene(s) in T cells *in vitro* of the invention, wherein the oligodeoxyribonucleic acid is a double-stranded DNA with a length of 100-250 bp or a single-stranded DNA with a length of 100 -250 nt.

In a preferred embodiment, in the method for knocking out one or more target gene(s) in T cells *in vitro* of the invention, wherein the crRNA targeting the TRAC gene is any one or more selected from the group consisting of the crRNAs shown in SEQ ID NOs: 1-12, the sequence of the crRNA targeting the B2M gene is shown in SEQ ID NO: 13, and the crRNA targeting the PD1 gene is any one or more selected from the group consisting of the crRNAs shown in SEQ ID NOs: 14-16.

In a preferred embodiment, in the method for knocking out one or more target gene(s) in T cells *in vitro* of the invention, wherein the Cas9 protein is a Cas9 protein derived from *Streptococcus pyogenes,* and the sequence thereof is shown in SEQ ID NO: 18.

In a preferred embodiment, in the method for knocking out one or more target gene(s) in T cells *in vitro* of the invention, wherein the Cas9 protein is a Cas9 protein derived from *Streptococcus pyogenes,* and the sequence of the tracrRNA corresponding to the Cas9 protein is shown in SEQ ID NO: 17.

In a preferred embodiment, in the method for knocking out one or more target gene(s) in T cells *in vitro* of the invention, wherein the T cell is selected from the group consisting of helper T cells, cytotoxic T cells, memory T cells, regulatory T cells, natural killer T cells, γδT cells, CAR-T cells, and TCR-T cells.

In another aspect, the present invention further provides T cells with target gene been knocked out, wherein the T cells are obtained by the method described above.

In another aspect, the invention further provides a crRNA for use in knocking out a target gene, wherein the crRNA comprises one or more sequences selected from the group consisting of SEQ ID NOs: 1-16.

In a preferred embodiment, provided is the crRNA used for knocking out a target gene out in the present invention targets a coding sequence of the target gene or targets a regulatory sequence for the expression of the target gene, wherein the target gene is one or more selected from the group consisting of TRAC, TRBC, B2M and PD1 genes.

In a preferred embodiment, provided is the crRNAs for use in knocking out a target gene in the present invention, wherein the target gene is TRAC gene, and the crRNA is one or more selected from the group consisting of those shown in SEQ ID NOs: 1-12.

In a preferred embodiment, provided is the crRNAs for use in knocking out a target gene in the present invention, wherein the target gene is B2M gene, and the sequence of the crRNA is set forth in SEQ ID NO: 13.

In a preferred embodiment, provided is the crRNAs for use in knocking out a target gene in the present invention, wherein the target gene is PD1 gene, and the crRNA is one or more selected from the group consisting of those shown in SEQ ID NOs: 14-16.

In another aspect, the present invention also provides a sgRNA for use in knocking out a target gene, the sgRNA consists of a crRNA linked to a tracrRNA corresponding to Cas9 protein, and wherein the crRNA comprises one or more sequences selected from the group consisting of SEQ ID NOs: 1-16.

In a preferred embodiment, provided is the sgRNAs for use in knocking out a target gene according to the present invention, wherein the target gene is one or more selected from the group consisting of TRAC, TRBC, B2M and PD1 genes.

In a preferred embodiment, provided is the sgRNAs for use in knocking out target gene TRAC according to the present invention, wherein the sgRNA consists of a crRNA linked to a tracrRNA corresponding to Cas9 protein, and the crRNA is one or more selected from the group consisting of those shown in SEQ ID NOs: 1-12.

In a preferred embodiment, provided is the sgRNAs for use in knocking out target gene B2M according to the present invention, wherein the sgRNA consists of a crRNA linked to a tracrRNA corresponding to Cas9 protein, and the crRNA is shown in SEQ ID NO: 13.

In a preferred embodiment, provided is the sgRNAs for use in knocking out target gene PD-1 according to the present invention, wherein the sgRNA consists of a crRNA linked to a tracrRNA corresponding to Cas9 protein, and the crRNA is one or more selected from the group consisting of those shown in SEQ ID NOs: 14-16.

In a preferred embodiment, provided is the sgRNAs for use in knocking out target gene according to the present invention, wherein the Cas9 protein is a Cas9 protein derived from *Streptococcus pyogenes,* with amino acid of SEQ ID NO: 18.

In a preferred embodiment, provided is the sgRNAs for use in knocking out target gene according to the present invention, wherein the sequence of the tracrRNA corresponding to the Cas9 protein is shown in SEQ ID NO: 17.

In another aspect, the invention provides a gene knockout kit, wherein the kit comprises:
a: one or more crRNA(s) as described above, or one or more sgRNA(s) as described above;
b: Cas9 protein;
c: an oligodeoxyribonucleic acid or a milt DNA fragment.

In a preferred embodiment, in the kit used for knocking out genes, the oligodeoxyribonucleic acid is a double-stranded DNA with a length of 100-250 bp or a single-stranded DNA with a length of 100-250 nt.

In a preferred embodiment, in the kit used for knocking out genes, the Cas9 protein is a Cas9 protein derived from *Streptococcus pyogenes,* and the sequence of the tracrRNA corresponding to the Cas9 protein is shown in SEQ ID NO: 17.

In some embodiments, the invention provides the use of T cells involving gene been knockout of the invention for the preparation of anti-tumor drugs.

In some embodiments, the present invention also provides use of the T cells involving gene been knockout of the invention for the preparation of a medicament for preventing or treating infectious diseases caused by viruses or bacteria.

In some embodiments, TCR, B2M or PD1 are effectively knocked out by using the designed crRNA and the method. The killing activity *in vitro* of CART cells with knocking out of TCR and B2M and/or PD1 gene(s), are not affected due to the knockout of TCR, B2M and/or PD1 genes.

### DESCRIPTION OF THE DRAWINGS

**Figure** 1: Comparison of knockout efficiencies of different delivery systems. The results show that the RNP delivery mode has the highest efficiency in knocking out genes in Jurkat cells.
**Figure** 2A-2B: Effect of N-oligo on efficiency of CRISPR-Cas9-based system in knocking out genes in T cells. Figure 2A shows a comparison of efficiency in knocking out genes in T cells; Figure 2B shows a comparison of efficiency in knocking out genes in CART cells.
**Figure** 3: Effect of milt DNA fragments on efficiency in gene knockout in T cells.
**Figure** 4: Detection of efficiency in B2M gene knocout.
**Figure** 5: Detection of the effect of the screened crRNAs on PD1 gene knockout.
**Figures** 6A-6B: Analysis of gene mutations caused by RNP and N-Oligo or milt DNA. Figure 6A shows analytic results for TRAC, and Figure 6B shows analytic results for B2M.
**Figures** 7A-7C: Analysis of RNP off-target rate. Figure 7A shows analytic results of the off-target rate of the TRAC gene; Figure 7B shows analytic results of the off-target rate of the B2M gene; and Figure 7C shows analytic results of the off-target rate of the PD1 gene.
**Figures** 8A-8B: Analysis of activation of CD25 and CD69 in T cells with TRAC gene knocked out. Figure 8A shows a comparison of CD69 activation; and Figure 8B shows a comparison of CD25 activation.

### DETAILED DESCRIPTION OF THE INVENTION

In one aspect, the invention provides a method for altering a target gene in a cell.

The study described herein demonstrates that the use of the allele targeting approach of CRISPR/Cas system to produce mutant cells with an efficiency up to 80%. In particular, the work described herein surprisingly and unexpectedly demonstrates a multiplex guide strategy that provides a method for specifically identifying useful RNA guide sequences, as well as specific guide sequences suitable for targeting specific genes (e.g., TRAC, TRBC, B2M, PD1).

CRISPR systems are used for the methods and compositions provided by the present invention, such systems also include those described in International Publication No. WO 2013142578 A1 and WO 2013098244 A1, which are incorporated herein entirely by reference.

An exemplary method for altering polynucleotide sequences of a target gene in a cell comprises contacting the polynucleotide sequences with a CRISPR (Clustered Regularly Interspaced Short Palindromic Repeats) sequence-related protein (Cas) and one or two ribonucleic acids, thereby a RNP is formed, wherein the ribonucleic acids direct the Cas protein to a target motif of the polynucleotide sequence of the target gene and to hybridize with the target motif, wherein the polynucleotide sequence of the target gene is cleaved, and the alternation efficiency of cells transformed with the RNP is 75% or more.

Any means for altering the polynucleotide sequence of a target gene is contemplated in the present invention, said means is readily available to the skilled artisan by using the CRISPR/Cas system of the present invention. Any CRISPR/Cas system capable of altering the polynucleotide sequence of a target gene in a cell can be used. Such CRISPR/Cas system can employ a variety of Cas proteins (Haft et al. PLoS Comput Biol. 2005; 1(6) e60). Such Cas proteins allow the CRISPR/Cas system to alter the polynucleotide sequence of the target gene in the cell, including RNA binding proteins, endonucleases and exonucleases, helicases, and polymerases. In some embodiments, the CRISPR/Cas system is a CRISPR Type I system. In some embodiments, the CRISPR/Cas system is a CRISPR Type II system.

The CRISPR/Cas system of the present invention can be used to alter the polynucleotide sequence(s) of a target gene in a cell. The present invention contemplates altering the polynucleotide sequence(s) of a target gene in a cell for any purpose. In some embodiments, the polynucleotide sequence(s) of a target gene in a cell is/are altered to produce a mutant cell.

An exemplary Cas9 protein herein may be a *Streptococcus pyogenes* Cas9 protein or a functional portion thereof. In some embodiments, the Cas9 protein is a Cas9 protein derived from any bacterial species or a functional portion thereof. The Cas9 protein is a member of the Type II CRISPR system, which typically includes a trans-encoded small RNA (tracrRNA), an endogenous ribonuclease 3 (rnc), and a Cas9 protein.

CrRNA (CRISPR-derived RNA) and tracrRNA (trans-activating RNA) are genetically engineered and are ligated together to obtain sgRNA (single guide RNA). Finally, a complex is formed by sgRNA-expressing sequence and Cas9 is transformed into a cell, thereby the target genes can be knocked out.

In some embodiments, such alteration corrects some undesired polynucleotide sequences of the target gene into desired sequences. The CRISPR/Cas system of the invention can be used to correct any type of mutation or error in the polynucleotide sequence of a target gene. For example, the CRISPR/Cas system of the invention can be used to insert the nucleotide sequences which have been missed from the polynucleotide sequences of a target gene due to deletion. In some examples, the CRISPR/Cas system of the present invention can also be used to delete or excise some nucleotide sequences caused by insertion mutation from the polynucleotide sequences of a target gene. In some embodiments, the CRISPR/Cas system of the invention can be used to replace incorrect nucleotide sequences with correct nucleotide sequences (e.g., to recover the impaired function due to the functional mutation of polynucleotide sequences of a target gene, i.e. SNP).

The CRISPR/Cas system of the present invention can unexpectedly cleave target genes with high efficiency, when compared with conventional CRISPR/Cas system. In certain embodiments, the efficiency of cleaving of the target gene is at least about 5%. In certain embodiments, the efficiency of cleaving of the target gene is at least about 10%. In certain embodiments, the efficiency of cleaving of the target gene is from about 10% to about 80%. In certain embodiments, the efficiency of cleaving of the target gene is from about 30% to about 80%. In certain embodiments, the efficiency of cleaving of the target gene is from about 50% to about 80%. In some embodiments, the efficiency of cleaving of the target gene is greater than or equal to about 75%, or greater than or equal to about 80%.

In some embodiments, the target gene is a genome. In some embodiments, the target gene is a human genome. In some embodiments, the target gene is a mammalian genome. In some embodiments, the target gene is a vertebrate genome.

The CRISPR/Cas system of the present invention can be applied in various applications to knock out the polynucleotide sequences or a portion thereof in the target gene. For example, knocking out the target gene polynucleotide sequences in a cell can be performed *in vitro* for research purposes. For *ex vivo* purpose, knocking out the target gene polynucleotide sequence(s) in a cell is applicable for treating or preventing disorders associated with the expression of the target gene polynucleotide sequence(s) (e.g., knocking out the mutant allele(s) in the cells *ex vivo*; and introducing those cells with mutant allele(s) been knockout back into the subject).

In another aspect, the invention provides a method for treating or preventing disorders associated with the expression of the polynucleotide sequences in a subject.

In order to understand the present invention easily, certain technical and scientific terms are specifically defined below. All of the other technical and scientific terms used herein have the meaning commonly understood by one of ordinary skill in the art to which this invention belongs, unless otherwise explicitly defined herein.

### I. Terminology

As used herein, the term "contacting" (i.e., contacting a polynucleotide sequences with a clustered regularly interspaced short palindromic repeats-associated (Cas) protein and/or ribonucleic acid) is intended to include incubating the Cas protein and/or ribonucleic acids in the cell together *in vitro* (e.g., adding the Cas protein or nucleic acid encoding the Cas protein to cells in culture) or contacting a cell *ex vivo.* The step of contacting the target gene polynucleotide sequence with a Cas protein and/or ribonucleic acid as disclosed herein can be conducted in any suitable manner. For example, the cells can be treated in adherent or suspension culture. It is understood that cells contacted with Cas protein and/or ribonucleic acid as disclosed herein may also be simultaneously or subsequently contacted with another agent, such as a growth factor or other differentiation agent or environment, to stabilize or to differentiate the cells further.

The term "treating" and the like, as applied to an isolated cell, include subjecting the cell to any kind of process or condition, or performing any kind of manipulation or procedure on the cell. As applied to a subject, the term refers to providing a cell in which a target gene polynucleotide sequence has been altered *ex vivo* according to the method described herein to an individual. The individual is typically suffered from diseases or injured, or is at increased risk of suffering from diseases relative to an average member of the population and in need of such attention, care or management.

As used herein, the term "treating" refers to administering to a subject an effective amount of cells with target polynucleotide sequences altered *ex vivo* according to the method described herein, so that the subject has a reduction in at least one symptom of the disease or an improvement in the disease, for example, beneficial or desired clinical results. For the purpose of the present invention, beneficial or desired clinical results include, but are not limited to, alleviation of one or more symptoms, diminishment of extent of the disease, stabilized state (i.e., i.e., not deterioration) of the disease, delay or slowing the disease progression, amelioration or palliation of the disease state, and remission (whether partial or total), whether detectable or undetectable. Treatment may refer to prolonging survival, as compared to the expected survival without treatment. Thus, one of skill in the art recognizes that a treatment may improve a disease condition, but may not be complete cure for the disease. As used herein, the term "treatment" includes prophylaxis. Alternatively, treatment is considered to be "effective" if the progression of a disease is reduced or halted. "Treatment" can also refer to prolonging survival as compared to expected survival without treatment. Those in need of treatment include those already diagnosed with a disorder associated with expression of the polynucleotide sequence, as well as those likely to develop such a disorder due to genetic susceptibility or other factors.

As used herein, a "mutant cell" refers to a cell with a resulting genotype that differs from the original genotype. In some examples, "a mutant cell" exhibits a mutant phenotype, for example, when a normally functioning gene is altered using the CRISPR/Cas system of the invention. In other examples, "a mutant cell" exhibits a wild-type phenotype, for example, when a mutated genotype is corrected by using the CRISPR/Cas system of the invention. In some embodiments, the target polynucleotide sequence(s) in a cell is/are altered to correct or repair the genetic mutation (e.g., to restore the normal genotype of the cell). In some embodiments, the target gene polynucleotide sequence(s) in a cell is/are altered to induce a genetic mutation (e.g., to destroy the function of a gene or genome element).

In some embodiments, the alteration is an indel. "Indel" as used herein refers to a mutation resulting from an insertion, deletion or combination thereof. As will be appreciated by those skilled in the art, an indel in the coding region of a genomic sequence will result in a frameshift mutation, unless the length of the indel is a multiples of three. In some embodiments, the alteration is a point mutation. "Point mutation" as used herein refers to the substitution of one of the nucleotides. The CRISPR/Cas system of the invention can be used to induce an indel of any length or point mutation in a target polynucleotide sequence.

"oligodeoxyribonucleic acid" or "N-oligo" refers to a deoxyribonucleic acid fragment with random sequence which has been transformed into a cell together with RNP, when RNP delivery system is used to knock out a gene. Preferably, it is a double-stranded DNA with 100-250 bp in length, or a single-stranded DNA with 100-250 nt in length.

"Milt DNA fragment" refers to small molecule fragments obtained by cutting milt DNAs by mechanically shearing a solution containing salmon sperm DNAs. For example, 1% salmon sperm DNAs solution is repeatedly beaten with a 7-gauge needle to cut DNAs into small molecules, aliquoted and stored.

"Knockout" as used herein includes deleting all or a portion of the target polynucleotide in a manner that interfers with the function of the target polynucleotide sequence. For example, a knockout can be achieved by altering a target gene polynucleotide sequence by inducing an indel in the functional domain (e.g., in the DNA binding domain) of the target polynucleotide sequence. Based on the details described herein, those skilled in the art will readily appreciate how to use the CRISPR/Cas systems of the present invention to knock out the target polynucleotide or a portion thereof.

In some embodiments, cleavage of the target gene results in a decreased expression of the target gene. The term "decreased" is generally used herein to mean a statistically significant decreased amount. However, for avoidance of doubt, "decreased" means a decreased by at least 10% as compared to a reference level, for example decreased by at least about 20%, or at least about 30%, or at least about 40%, or at least about 50%, or at least about 60%, or at least about 70%, or at least about 75%, or at least about 80%, or at least about 90%, or up to and including a 100% decrease (i.e., absent level as compared to the a reference sample), or any decreased between 10% and 100% as compared to a reference level.

The term "statistically significant" or "significantly" refers to statistical significance and generally means a two standard deviation (2SD) below normal, or lower, concentration of the marker. The term refers to statistical evidence for the presence of difference. It is defined as a probability of making a decision to reject the null hypothesis when the null hypothesis is actually true. The decision is often made using the p-value.

In some embodiments, cleavage of the target gene is cleavage of a homozygous target gene. In some embodiments, cleavage of the target gene is cleavage of a hybrid target gene.

Cas9 protein (also known as CRISPR-related endonuclease Cas9/Csn1) is a polypeptide comprising 1368 amino acids. An exemplary amino acid sequence of a Cas9 protein is shown in SEQ ID NO: 18. Cas9 contains two endonuclease domains, including the RuvC-like domain (residues 7-22, 759-766, and 982-989), which cleaves target DNA is noncomplementary to crRNA; and an HNH nuclease domain (residues 810-872), which cleaves target DNA complementary to crRNA.

T cell receptor (TCR) is a hetero-dimeric protein receptor that presents a specific antigenic peptide on the Major Histocompatibility Complex (MHC). In the immune system, the binding of antigen-specific TCR to the pMHC complex triggers direct physical contact between T cells and Antigen Presenting Cells (APCs), and then the other cell membrane surface molecules on T cells interact with those on APCs. This leads to a series of subsequent cell signaling and other physiological responses, allowing different antigen-specific T cells to exert an immune effect on their target cells.

TCR is a glycoprotein present on cell membrane surface in the form of heterodimer of alpha chain/beta chain or gamma chain/delta chain. The TCR heterodimer in 95% of T cells consists of alpha and beta chains, while 5% of T cells have a TCR consisting of gamma and delta chains. The native αβ heterodimeric TCR has alpha chain and beta chain, and the alpha chain and the beta chain constitute the subunit of the αβ heterodimeric TCR. Broadly, each of the alpha and beta chains comprises a variable region, a junction region, and a constant region, and the beta chain typically also contains a short diversity region between the variable region and the junction region, but the diversity region is often considered as a part of the junction region. Each variable region comprises three CDRs (complementarity determining regions), CDR1, CDR2 and CDR3, which are interspersed among the framework regions. The CDR regions determine the binding of the TCR to the pMHC complex, wherein the CDR3 is recombinantly formed by the variable region and the junction region and is referred to as the hypervariable region. The alpha and beta chains of TCR are generally considered to have two "domains", namely a variable domain and a constant domain, and the variable domain consists of the variable region linked to the junction region. The constant domain sequence of the TCR can be found in the public database of the International Immunogenetics Information System (IMGT). For example, the constant domain sequence of α chain of the TCR molecule is "TRAC^{∗}01", and the constant domain sequence of β chain of the TCR molecule is "TRBC1^{∗} 01" or "TRBC2^{∗}01". In addition, the alpha and beta chains of TCR also contain transmembrane and cytoplasmic regions, wherein the cytoplasmic region is very short.

B2M, also known as beta-2 microglobulin, is the light chain of MHC class I molecules and is therefore an indispensable part of MHC. In human, B2M is encoded by b2m gene located on chromosome 15, and opposite to other MHC genes as a cluster of genes located on chromosome 6. The protein derived from human consists of 119 amino acids and has a molecular weight of 11,800 Daltons. A murine model with β-2 microglobulin deficiency has demonstrated that B2M is necessary for expressing MHC class I on cell surface and for the stability of peptide-binding cleft.

"PD-1" or "PD1" is a type I transmembrane receptor with 50-55 kDa, which was originally identified in T cell suffering from activation-induced apoptosis. PD-1 is expressed on T cells, B cells and macrophages. The ligands of PD-1 are the members of B7 family, PD-L1 (B7-H1) and PD-L2 (B7-DC).

PD-1 is a member of the immunoglobulin (Ig) superfamily and contains a single IgV-like domain in its extracellular region. The PD-1 cytoplasmic domain contains two tyrosines, the one that is closer to membrane (VAYEEL in mouse PD-1) is located within ITIM (the inhibitory motif of the immunoreceptor tyrosine). The ITIM on PD-1 predicts that this molecule acts by recruiting cytosolic phosphatase to attenuate the signaling of antigen receptors. The human and murine PD-1 proteins share approximately 60% amino acid identity, with four potentially conserved N-glycosylation sites and residues defining the Ig-V domain. The cytoplasmic ITIM and the ITIM-like motif around the carboxy terminal tyrosine (TEYATI in human and mice) are also conserved between human and murine orthologues.

### II. Examples and Test Examples

The invention is further described in the following examples, but these examples are not intended to limit the scope of the invention.

Experimental methods, for which the specific conditions are not specifically indicated in the examples or test examples of the present invention, were generally carried out according to conventional conditions or according to the conditions recommended by the manufacturers. Reagents, for which the sources are not specifically indicated, were routinely purchased from the market.

### EXAMPLES

### Example 1: PBMC Extraction

Healthy volunteers with no cold and fever symptom were recruited, and informed consents were signed. 100 ml of blood was taken from vein into a BD anticoagulant tube by medical professionals. The blood was mixed with an equal amount of PBS buffer (containing 2% fetal bovine serum). 15 ml of Ficoll buffer (GE healthcare) was added into PBMC separation tube Sepmate-50 (STEMCELL Technology), and the mixture of blood and PBS was then added. The pellet was resuspended in PBS after centrifugation. The resuspended cells were counted, 10 µl of the suspension was added with 10 µl of 0.1% trypan blue, mixed; cell numbers and survival rates were counted.

### Example 2: Purification of T Cells

PBMCs were centrifuged at 300 g for 5 minutes, the supernatant was discarded, and added with corresponding amount of PBS buffer (containing 2 mM EDTA and 1% fetal bovine serum), the cells were resuspended and the cell density was adjusted to 5 × 10⁷/ml. Human T cells were purified by using EasySep™ Human T Cell Enrichment Kit available from STEMCELL Technology. First, 50µl/ml of Cooktail was added to the PBMC suspension, mixed well and placed at room temperature for 10 minutes. Then, 50µl/ml of EasySep™ D Magnetite Particles were added, mixed well, and placed at room temperature for 5 minutes. The cell suspension was added to a 5 ml flow tube and placed in magnetic poles for 5 minutes. The cell suspension was quickly decanted, PBS buffer was added into the flow tube and resuspended, repeated 3 times. The obtained cell suspension was centrifuged at 300 g for 5 minutes, the supernatant was discarded, and the cell pellets were resuspended in VIVO-15 medium available from LONZA, the density was adjusted to 1 × 10⁶/ml, and rIL-2 (R&D) was added to make the concentration to reach 100 IU/ml. Then, it was cultured in 37°C cell culture incubator.

### Example 3: T Cell Activation

Anti-CD3/anti-CD28 magnetic beads (Life Technology) were resuspended in PBS buffer (containing 2 mM EDTA and 1% fetal bovine serum), then placed in magnetic poles for 2 minutes, and then the supernatant was discarded. The above process was repeated 4 times. The magnetic beads were washed, and then added into the purified T cells in a ratio of 1:1, mixed and cultured at 37°C for 3 days. After 3 days, the magnetic beads were removed, and the target cells were resuspended several times by pipetting. The cell suspension was placed in magnetic poles, placed for two minutes, and the magnetic beads on the tube wall were discarded.

### Example 4: Infecting T Cells with CAR Virus

Construction of CAR lentiviral Plasmid:
The structure of CD19 CAR from the outside to the inside is CD19 scFv, Hinge structure, transmembrane structure, 4-1BB and CD3z. An expression vector was constructed with CD19 CAR and vector pHR-CAR. The lentiviral plasmid pHR-CAR and the two helper plasmids dR8.91 and pCMV-VSV-G were extracted by using Plasmid Extraction maxi Kit available from Tiangen.

Package and Concentration of CAR Lentivirus:
293T cells (purchased from ATCC) were overgrown on a 75 cm² culture dish one day before transfection, and passaged at 1:3 with 15 ml of culture medium for each dish. Transfection was carried out according to the procedure of Lipo3000. The transfection system was as follows:

| Transfection system 1 | Transfection system 2 |
|---|---|
| pHR-CAR: 7.5/µg | |
| dR8.91: 5.625µg | |
| pCMV-VSV-G: 1.875µg | |
| Opti-MEM (Gibco): 700µl | Opti-MEM (Gibco): 700µl |
| P3000: 30µl | Lipofectamine: 36µl |

The systems 1 was mixed well with the system 2, placed for 5 minutes and mixed again, and then placed for another 10 minutes. 293T cells were carefully added. 6 hours later, the medium was replaced with fresh medium. 48 hours later, the culture medium was collected and stored at 4 °C. 15 ml of fresh medium was added again, and the supernatant was collected 24 hours later. The obtained virus supernatant was filtered through 0.45 µm filter and added in ultracentrifuge tube. The tubes were centrifuged at 50000g for 2 hours and 45 minutes at 4°C, the supernatant was carefully and thoroughly removed, and the white virus pellet visible to naked eyes was resuspended with PBS buffer at a volume of 1% of the supernatant volume. The resuspended virus was dissolved at 4 °C for about 30 minutes. After completely dissolved, it was aliquoted and stored in a refrigerator at -80 °C.

Infecting T Cells with CAR Lentivirus:
The human primary T cells were activated with anti-CD3/anti-CD28 magnetic beads, one day later, the cells were resuspended, placed in magnetic poles for two minutes, the cell suspension was taken and cell counting was performed. About 1 × 10⁷ cells were centrifuged at 300 g for 5 minutes, the medium was discarded, and 1 ml of fresh medium was added to resuspend the cells. Concentrated lentivirus was added to adjust the MOI to 5, mixed well. The tubes were centrifuged at 2000 g for 90 minutes at 32 °C, the supernatant was discarded, and fresh medium (100 IU/ml rIL-2) was added to adjust the cell density to 1 × 10⁶ cells/ml, the cells were resuspended and added with the newly isolated anti-CD3/anti-CD28 magnetic beads. The cultivation was continued in an incubator at 37 °C. CAR-T cells were obtained.

### Example 5: Knockout of TCR, B2M, PD1 Gene

### (1) Design of crRNA

An appropriate target region was selected based on the nucleotide sequences of TRAC, B2M and PD1, and a crRNA with length of 17-20 nt was designed. A sgRNA was formed by ligating the crRNA with a tracrRNA corresponding to Cas9 protein used herein. The crRNAs with high knockout efficiency and low off-target rate were screened by experiments. The sequences of the selected crRNAs were as follows:

**Table 1 crRNAs against target genes**

| Target gene | crRNA name | sequence | SEQ ID NO. |
|---|---|---|---|
| TRAC | crRNA-1 | CAAAUGUGUCACAAAGUA | 1 |
| | crRNA-2 | AAAACUGUGCUAGACAUG | 2 |
| | crRNA-3 | UCAAGAGCAACAGUGCUG | 3 |
| | crRNA-4 | CACCUUCUUCCCCAGCCC | 4 |
| | crRNA-5 | GAAUAAUGCUGUUGUUGA | 5 |
| | crRNA-6 | GAUUUAGAGUCUCUCAGC | 6 |
| | crRNA-7 | ACGGCAGGGUCAGGGUUC | 7 |
| | crRNA-8 | GUUCCUGUGAUGUCAAGC | 8 |
| | crRNA-9 | UCAAAACCUGUCAGUGAU | 9 |
| | crRNA-10 | GAAUCCUCCUCCUGAAAG | 10 |
| | crRNA-11 | GGUACACGGCAGGGUCA | 11 |
| | crRNA-12 | GAGAAUCAAAAUCGGUGAAU | 12 |
| B2M | crRNA-13 | GUAGCGCGAGCACAGCUA | 13 |
| PD1 | crRNA-14 | CGACUGGCCAGGGCGCCUGU | 14 |
| | crRNA-15 | GUGCUACAACUGGGCUGG | 15 |
| | crRNA-16 | GGCGCCCUGGCCAGUCGUCU | 16 |

Cas9 protein is derived from *Streptococcus Pyogenes* (Cas9 Nuclease NLS, *S. Pyogenes* (BioLabs)), the corresponding tracrRNA sequence (SEQ ID NO: 17) was:

GUUUUAGAGCUAGAAAUAGCAAGUUAAAAUAAGGCUAGUCCGUUAUCAA
CUUGAAAAAGUGGCACCGAGUCGGUGCUUUU .

The amino acid sequence of the Cas9 (including NLS) protein used herein (SEQ ID NO: 18):

MDKKYSIGLAIGTNSVGWAVITDEYKVPSKKFKVLGNTDRHSIKKNLIGAL
LFDSGETAEATRLKRTARRRYTRRKNRICYLQEIFSNEMAKVDDSFFHRLEESFL
VEEDKKHERHPIFGNIVDEVAYHEKYPTIYHLRKKLVDSTDKADLRLIYLALAH
MIKFRGHFLIEGDLNPDNSDVDKLFIQLVQTYNQLFEENPINASGVDAKAILSAR
LSKSRRLENLIAQLPGEKKNGLFGNLIALSLGLTPNFKSNFDLAEDAKLQLSKDT
YDDDLDNLLAQIGDQYADLFLAAKNLSDAILLSDILRVNTEITKAPLSASMIKRY
DEHHQDLTLLKALVRQQLPEKYKEIFFDQSKNGYAGYIDGGASQEEFYKFIKPIL
EKMDGTEELLVKLNREDLLRKQRTFDNGSIPHQIHLGELHAILRRQEDFYPFLKD

NREKIEKILTFRIPYYVGPLARGNSRFAWMTRKSEETITPWNFEEVVDKGASAQS
FIERMTNFDKNLPNEKVLPKHSLLYEYFTVYNELTKVKYVTEGMRKPAFLSGEQ
KKAIVDLLFKTNRKVTVKQLKEDYFKKIECFDSVEISGVEDRFNASLGTYHDLL
KIIKDKDFLDNEENEDILEDIVLTLTLFEDREMIEERLKTYAHLFDDKVMKQLKR
RRYTGWGRLSRKLINGIRDKQSGKTILDFLKSDGFANRNFMQLIHDDSLTFKEDI
QKAQVSGQGDSLHEHIANLAGSPAIKKGILQTVKVVDELVKVMGRHKPENIVIE
MARENQTTQKGQKNSRERMKRIEEGIKELGSQILKEHPVENTQLQNEKLYLYYL
QNGRDMYVDQELDINRLSDYDVDHIVPQSFLKDDSIDNKVLTRSDKNRGKSDN
VPSEEVVKKMKNYWRQLLNAKLITQRKFDNLTKAERGGLSELDKAGFIKRQLV
ETRQITKHVAQILDSRMNTKYDENDKLIREVKVITLKSKLVSDFRKDFQFYKVR
EINNYHHAHDAYLNAVVGTALIKKYPKLESEFVYGDYKVYDVRKMIAKSEQEI
GKATAKYFFYSNIMNFFKTEITLANGEIRKRPLIETNGETGEIVWDKGRDFATVR
KVLSMPQVNIVKKTEVQTGGFSKESILPKRNSDKLIARKKDWDPKKYGGFDSPT
VAYSVLVVAKVEKGKSKKLKSVKELLGITIMERSSFEKNPIDFLEAKGYKEVKK
DLIIKLPKYSLFELENGRKRMLASAGELQKGNELALPSKYVNFLYLASHYEKLK
GSPEDNEQKQLFVEQHKHYLDEIIEQISEFSKRVILADANLDKVLSAYNKHRDKP
IREQAENIIHLFTLTNLGAPAAFKYFDTTIDRKRYTSTKEVLDATLIHQSITGLYET
RIDLSQLGGDPKKKRKVMDK
.

The sgRNA consisting of the crRNA shown in Table 1 linked to tracrRNA corresponding to Cas9 protein described above was prepared, wherein the crRNA was located at 5' end of the tracrRNA.

### (2) In vitro transcription of sgRNA:

PCR amplification of sgRNA template was performed first:

| Reaction System (20µl) | | PCR condition | |
|---|---|---|---|
| plasmid | 1 µl (0.5pg) | 98 °C | 30sec |
| 5×HF buffer | 4 µl | 98 °C | 10sec |
| dNTP (10mM) | 0.4µl | 60 °C | 25sec |
| primer-F(10µm) | 0.4 µl | 72 °C | 2min |
| primer-R(10µm) | 0.4 µl | 72 °C | 10min |
| Phusion | 0.2 µl | 38 times | |
| H₂O | 13.6 µl | 4°C | 10min |

Then, PCR products were recovered:
The Manual for Regular DNA Product Purification Kit DP214 was available from Tiangen and was used as a reference. DNA for *in vitro* sgRNA transcription was obtained. The sgRNA was transcribed with *in vitro* transcription kit MEGAshortscript™ Kit (Cat# AM1354) available from Ambion. The manual for Ambion MEGAclear™ Kit cat#AM1908 was used as a reference. The obtained sgRNAs were purified and detected by spectrophotometer and denatured agarose gel electrophoresis, all of them were qualified and aliquoted immediately for use.

### (3) TRAC gene was knocked out in T cells via electroporation of CRISPR-Cas9:

The obtained CAR-T cells were electro-transformed (this method is also applied to knocking out primary T cells), mainly using the LONZA 4D electroporation instrument, and the kit used was P3 Primary Cell 4D-Nucleofector™ X kit.

First, the electroporation system was prepared: 10 µl of Nucleofector buffer, 30 µg of Cas9 protein (about 9 µg/µl) and 4 µg of sgRNA were mixed and incubated at room temperature for 10 minutes. CAR-T cells were activated for three days, and then the anti-CD3/anti-CD28 magnetic beads were removed via magnetic poles. 5 × 10⁶ cells/tube were taken and centrifuged at 300 g for 5 minutes to completely remove the supernatant. The incubated electroporation system was added to the cell pellet, further added with 72 µl of Nucleofector buffer and 18 µl of Supplement buffer, mixed well and added to 100 µl of LONZA electroporation cuvette, which was placed in LONZA-4D electroporation instrument and electroporation was carried out according to the E0-115 procedure. After the electroporation was completed, the electroporation cuvette was allowed to stand at room temperature for 5 minutes. The cells were transferred from the electroporation cuvette into the pre-warmed VIVO-15 medium, cell density was adjusted to 1 × 10⁶ cells/ml, and cultured at 37 °C.

### Example 6: Screening of TCR-Negative Cells

After TRAC was knocked out from CAR-T cells with CRISPR-Cas9, the CAR-T cells were cultured until day 10, and TCR-negative cells were enriched. First, all of cells were centrifuged at 300g for 5 minutes, washed twice with PBS buffer (containing 2 mM EDTA and 1% fetal bovine serum). The cell density was adjusted to 1 × 10⁷ cells/ml, and then added with 100µl/ml of Biotin-TCR antibody (purchased from Miltenyi Biotec, Germany), and incubated for 10 minutes at 4°C in the darkness. After washing once with PBS buffer, the cell density was adjusted to 1 × 10⁷ cells/ml, Anti-Biotin Microbeads were added at 50 µl/ml, and kept at 4 °C for 15 minutes in the darkness. After washing once with PBS buffer, the cells were resuspended in 500 µl of buffer. LD column (purchased from Miltenyi Biotec) was placed in magnetic poles and rinsed with 2 ml of PBS buffer once. Then, 500 µl of the cell suspension was added, the target cells flowed through the LD column and were collected from the bottom of the LD column. When all of the cell suspension flowed out, 2 ml of PBS buffer was loaded onto the LD column, repeated twice. The collected cell suspension was centrifuged at 300 g for 5 minutes, and resuspended in pre-warmed medium.

### Test Examples

### Test Example 1: The most optimized crRNA was chosen for CRISPR-Cas9-based knockout of TRAC

The crRNA sequences designed for TRAC as indicated in Example 5 were compared in the test. sgRNA was obtained after *in vitro* transcription, and then Cas9 protein was electroporated into the activated primary T cells. 48 hours later, the extracellular expression of TCR protein was detected by flow cytometer. The results showed that all of crRNAs can knock out TRAC gene to varying degrees, among them, crRNA-11 exhibited the highest knockout efficiency.

### Test Example 2: Comparative Analysis of Different Delivery Systems

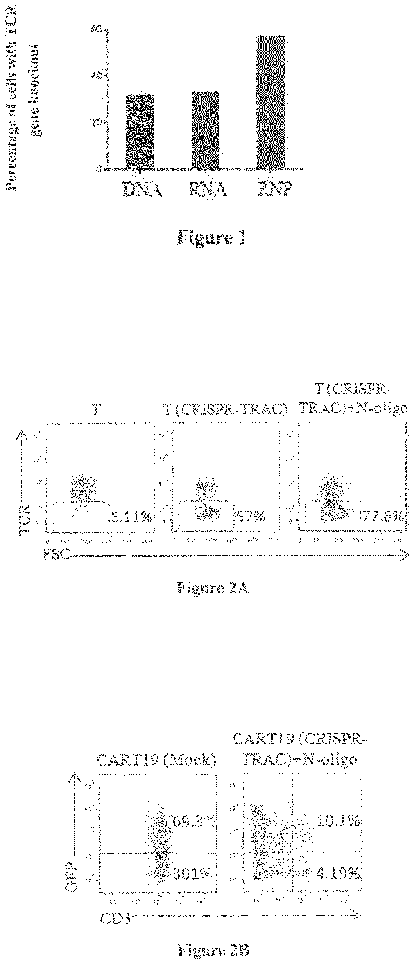

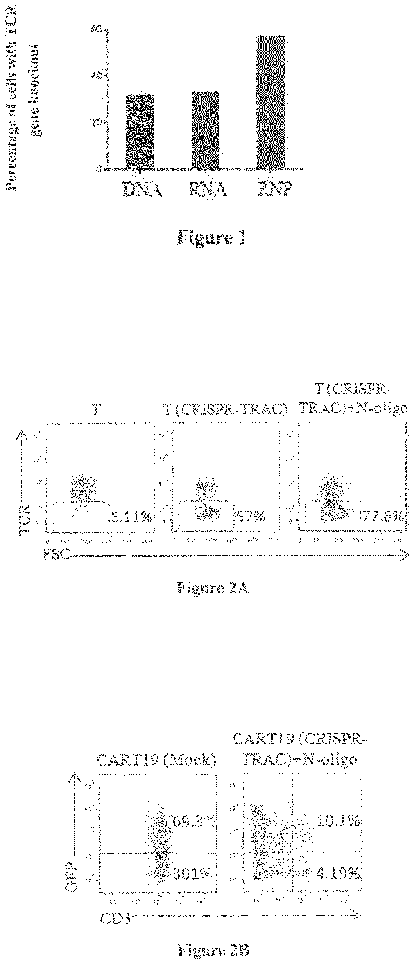

Three delivery systems were plasmid, mRNA and RNP (protein-RNA complex), respectively; crRNA was designed to target TRAC, and maxi plasmid extraction was performed according to Example 4. For *in vitro* transcription of Cas9 mRNA, DNA template containing T7 promoter was firstly obtained by PCR with T7 primers; then, the Cas9 mRNA was obtained by *in vitro* transcription with T7 *in vitro* Transcription Kit available from Ambion. sgRNA and Cas9 protein complex were obtained in the same way as those indicated in Example 5. 5×10⁶ Jurkat cells were centrifuged and the supernatant was discarded. Electroporation was performed with three different delivery materials respectively on Electroporation System Neon MPK5000 (Invitrogen). 48 hours later, 0.5 × 10⁶ cells were taken and washed twice with PBS buffer, resuspended with 100 µl of buffer, and then added with 10 µl of PE-TCR antibody (eBioscience), mixed well and incubated at 4 °C for 30 minutes. After washing once using PBS buffer, the cells were resuspended with 500 µl of buffer and loaded onto TCR detection channel of flow cytometer. The results were shown in Figure 1.

### Test Example 3: Random N-oligo or milt DNA increases the efficiency of CRISPR-Cas9 in knocking out TRAC

When RNP delivery system was used to knock out a gene, RNP was mixed well with random sequence of N-oligo (oligodeoxyribonucleic acid) or milt DNA and simultaneously transformed by electroporation.

An exemplary sequence of N-oligo:

TCATGTGGTCGGGGTAGCGGCTGAAGCACTGCACGCCGTACGTCAGGGTGGT
CACGAGGGTGGGCCAGGGCACGGGCAGCTTGCCGGTGGTGCAGATGAACTT
CAGGGTCAGCTTGCCGTAGGTGGC (SEQ ID NO: 19) .

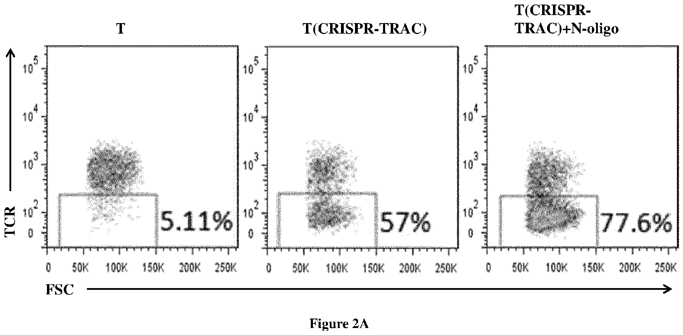

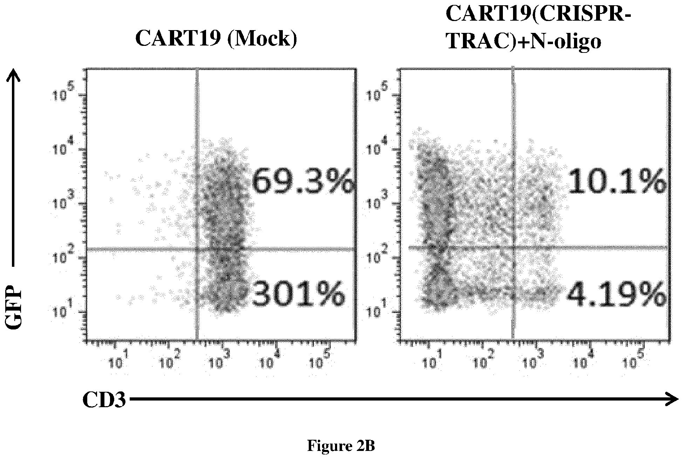

On the basis of Example 5 (3), 100-200 nM of N-oligo DNA was additionally added to the RNP complex, wherein the N-oligo DNA was Page-grade. The effect of N-oligo on the efficiency of CRISPR-Cas9 in knocking out TRAC was shown in Figure 2A- Figure 2B. The results showed that N-oligo can effectively increase the efficiency of CRISPR-Cas9 in knocking out TRAC gene in both T cells and CAR-T cells.

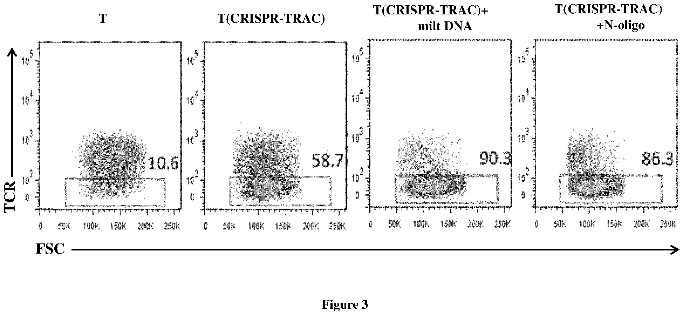

On the basis of Example 5 (3), 100-200 nM milt DNA fragments were additionally added to the RNP complex, and the effect of the milt DNA fragments on the efficiency in knocking TRAC out was shown in Figure 3. The results showed that the milt DNA fragments increased the efficiency of knocking out TRAC gene, and the efficiency is higher than that of N-oligo.

### Test Example 4: Detection of efficiency in knocking out B2M, PD1 in T cells

Similarly, a number of crRNAs were designed, and after comparative experiment, the crRNA with the highest knockout efficiency and the lowest off-target rate was chosen for knocking out B2M gene. B2M and/or PD1 genes were knocked out of T cells by using the RNP delivery system and N-oligo, based on the same method as that described in Example 5 (3).

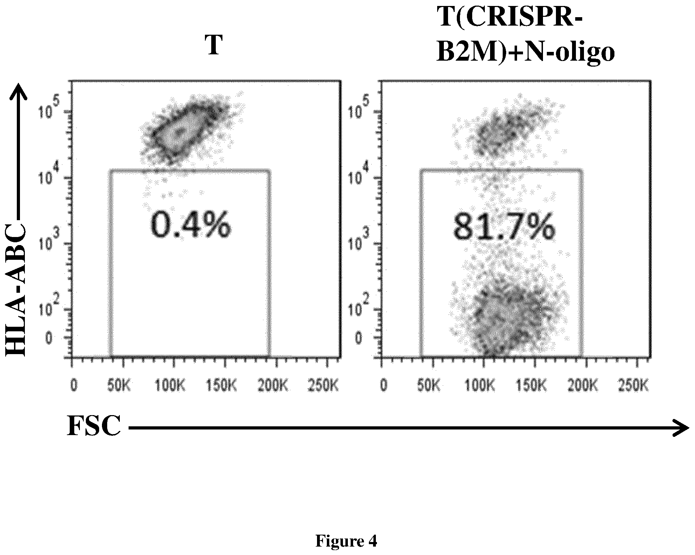

For B2M protein, the efficiency in knocking out B2M gene was detected by using APC-HLA-ABC antibody (eBioscience), because the expression of B2M gene was closely related to the display of HLA-ABC on cell membrane. The results (shown in Figure 4) showed that the efficiency in knocking out B2M gene was greater than 80%.

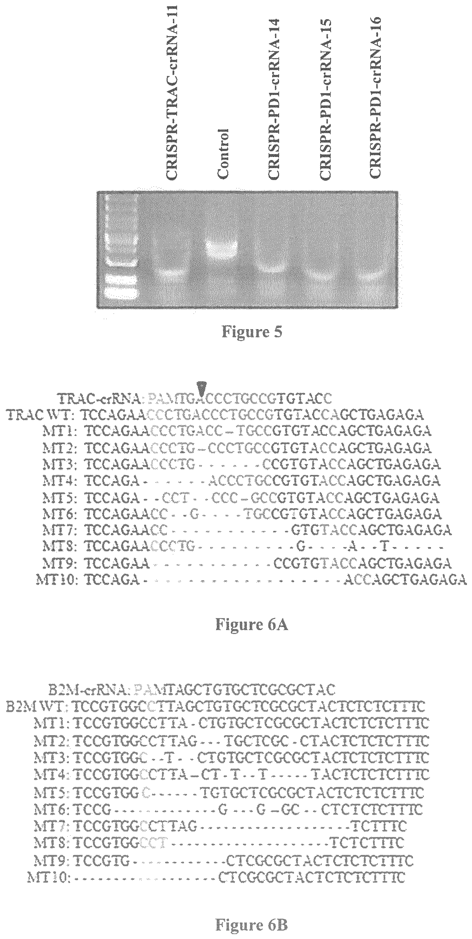

For PD1 gene, the cells were electroporated with the mixture of RNP and N-oligo for 48 hours, and 1×10⁶ cells were taken, washed twice with PBS buffer, and the supernatant was completely aspirated. T7E1 experiment was performed according to the manual of GeneArt® Genomic Cleavage Detection Kit (Thermo Fisher), and the results (shown in Figure 5) showed that the three crRNAs selected for PD1 were all able to effectively knock out PD1 gene, and the knockout efficiencies were all greater than 80%.

### Test Example 5: Analysis of gene mutations caused by CRISPR-Cas9

Primers were firstly designed near the target sites in TRAC, B2M and PD1 genes. After TRAC, B2M and PD1 were knocked out in T cells based on the CRISPR-Cas9 system, using RNP+N-oligo or milt DNA fragments, genomic DNAs were extracted from 1×10⁶ of both normal T cells and T cells in which gene has been knocked out, respectively. The obtained PCR product DNA fragment were ligated to a vector with T-blunt ends (pEASY-Blunt Simple Cloning Kit, Beijing TransGen Biotech Co., Ltd.), then the obtained vector was transformed into TOP10 competent cells which were plated onto the Amp-resistant solid plates. The next day, the obtained clones were sequenced, and at least 30 clones per plate were sequenced. The obtained sequencing results were aligned to the wild type sequence, and the results (as shown in Fig. 6A to Fig. 6B, the results for PD1 mutation were not shown) showed that CRISPR-Cas9 resulted in gene mutations in three genes at the genomic DNA sites corresponding to the crRNA, respectively.

### Test Example 6: Analysis of Off-target

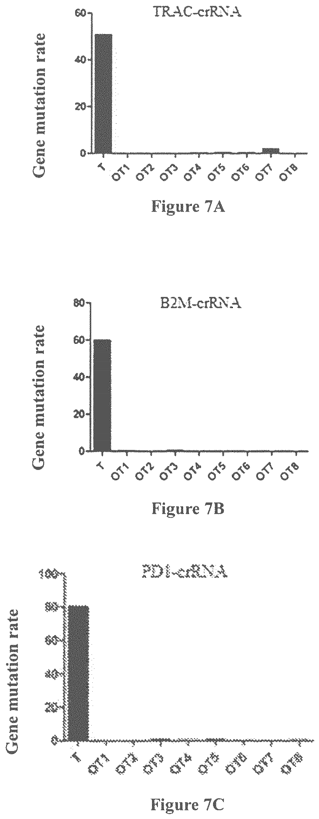

The possible off-target sites were predicted at http://crispr.mit.edu/, based on the designed crRNA. 8 or 9 potential off-target sites (OT1-OT9) were selected for TRAC, B2M and PD1, respectively. Primers were designed for these potential off-target sites for PCR amplification and sequencing. The peak mapping sequencing results for off-target sites of the genomic DNA knocked out in cells and for controls (target gene TRAC, B2M or PD1) were aligned by TIDE on website https://tide.nki.nl/, and the results were as shown in Figures 7A to 7C, indicating the off-target rate of the selected crRNA and the knockout method was extremely low.

### Test Example 7: Analysis of the effect of TCR knockout on both signaling pathway and killing activity of the cells

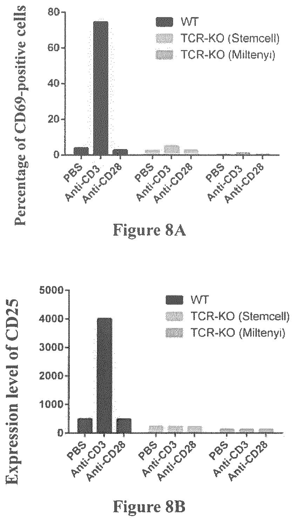

CD3 antibody solution (5 µg/ml) was prepared and coated on 96-well plate. A volume of 100 µl was added to each well, and coated at 37 °C for two hours. The plated was taken out and washed twice with PBS. TCR-negative T cells and normal T cells were added respectively, and the cell density was 1 × 10⁶ cells/ml. After incubation at 37 °C for 24 hours, the plate was taken out and stained with antibodies specific for CD25 and CD69, and the results (as shown in Figure 8A and Figure 8B) showed that T cells in which TRAC gene was knocked out cannot be induced to express CD25 and CD69 by CD3 antibody. Comparison of the killing effects of TCR(against CD19)-positive CAR-T and TCR-negative (such as knockout of TRAC) CAR-T cells on CD19-positive tumor cell line K562-CD19 showed that TCR knockout does not significantly impact the killing effect of CAR-T.

For clear understanding, the invention has been described in detail with the aid of the drawings and examples, however the description and examples should not be construed as limiting the scope of the invention. The disclosures of all patents and scientific literatures cited herein are expressly incorporated by reference in their entirety.

## Claims

1. A method for knocking out one or more target gene(s) in T cells *in vitro,* comprising the steps of:
1) contacting sgRNAs targeting one or more target gene(s) in the T cells with Cas9 protein, respectively, to form a protein-RNA complex;
2) mixing the protein-RNA complex with an oligodeoxyribonucleic acid or a milt DNA fragment; and transforming the obtained mixture into T cells, wherein the sgRNAs direct the Cas9 protein to the corresponding target sequence of the target gene and to hybridize with the target sequence, thereby the target gene is cleaved, and the cleavage efficiency of the target gene is greater than 75%.

2. The method for knocking out one or more target gene(s) in T cells *in vitro* according to claim 1, wherein the target gene is one or more selected from the group consisting of TRAC, TRBC, B2M and PD1 genes, and the sgRNA targets a coding sequence of the target gene or a regulatory sequence for the expression of the target gene.

3. The method for knocking out one or more target gene(s) in T cells *in vitro* according to claim 1 or 2, wherein the sgRNA consists of, from 5' to 3', a crRNA targeting the target gene with 17-20 nt in length, linked to a tracrRNA corresponding to the Cas9 protein.

4. The method for knocking out one or more target gene(s) in T cells *in vitro* according to any one of claims 1-3, wherein the oligodeoxyribonucleic acid is a double-stranded DNA with a length of 100-250 bp or a single-stranded DNA with a length of 100-250 nt.

5. The method for knocking out one or more target gene(s) in T cells *in vitro* according to any one of claims 1-4, wherein:
the crRNA targeting the TRAC gene is any one or more selected from the group consisting of crRNA as shown in SEQ ID NOs: 1-12,
the crRNA targeting the B2M gene is shown as SEQ ID NO: 13, and
the crRNA targeting the PD1 gene is any one or more selected from the group consisting of crRNA as shown in SEQ ID NOs: 14-16.

6. The method for knocking out one or more target gene(s) in T cells *in vitro* according to any one of claims 1-5, wherein the Cas9 protein is a Cas9 protein derived from *Streptococcus pyogenes,* with amino acid sequence of SEQ ID NO: 18.

7. The method for knocking out one or more target gene(s) in T cells *in vitro* according to any one of claims 1-6, wherein the sequence of the tracrRNA corresponding to the Cas9 protein is shown in SEQ ID NO: 17.

8. The method for knocking out one or more target gene(s) in T cells *in vitro* according to any one of claims 1-7, wherein the T cell is selected from the group consisting of helper T cell, cytotoxic T cell, memory T cell, regulatory T cell, natural killer T cell, γδT cells, CAR-T cell and TCR-T cell.

9. T cells with target gene knockout, wherein in the T cells are obtained by the method according to any one of claims 1-8.

10. A crRNA for use in knockout of a target gene, wherein the crRNA comprises one or more sequences selected from the group consisting of SEQ ID NOs: 1-16.

11. The crRNA for use in knockout of a target gene according to claim 10, wherein the target gene is one or more selected from the group consisting of TRAC, TRBC, B2M and PD1 genes.

12. The crRNA for use in knockout of a target gene according to claim 10 or 11, wherein the target gene is TRAC gene, and the crRNA is one or more selected from the group consisting of those shown in SEQ ID NOs: 1-12.

13. The crRNA for use in knockout of a target gene according to claim 10 or 11, wherein the target gene is B2M gene, and the sequence of the crRNA is shown in SEQ ID NO: 13.

14. The crRNA for use in knockout of a target gene according to claim 10 or 11, wherein the target gene is PD1 gene, and the crRNA is one or more selected from the group consisting of those shown in SEQ ID NOs: 14-16.

15. An sgRNA for use in knockout of a target gene, wherein the sgRNA consists of a crRNA linked to a tracrRNA corresponding to Cas9 protein, and wherein the crRNA comprises one or more sequences selected from the group consisting of SEQ ID NOs: 1-16.

16. The sgRNA for use in knockout of a target gene according to claim 15, wherein the target gene is one or more selected from the group consisting of TRAC, TRBC, B2M and PD1 genes.

17. The sgRNA for use in knockout of a target gene according to claim 15 or 16, wherein the target gene is TRAC gene, and the crRNA is one or more selected from the group consisting of those shown in SEQ ID NOs: 1-12.

18. The sgRNA for use in knockout of a target gene according to claim 15 or 16, wherein the target gene is B2M gene, and the crRNA is shown in SEQ ID NO: 13.

19. The sgRNA for use in knockout of a target gene according to claim 15 or 16, wherein the target gene is PD1 gene, and the crRNA is one or more selected from the group consisting of those shown in SEQ ID NOs: 14-16.

20. The sgRNA for use in knockout of a target gene according to claim 15, wherein the Cas9 protein is a Cas9 protein derived from *Streptococcus pyogenes,* with amino acid sequence of SEQ ID NO: 18.

21. The sgRNA for use in knockout of a target gene according to the claim 15, wherein the sequence of the tracrRNA corresponding to the Cas9 protein is shown in SEQ ID NO: 17.

22. A kit for use in gene knockout, wherein the kit comprises:
a: one or more crRNA(s) according to any one of claims 9-14, or one or more sgRNAs according to any one of claims 15-21;
b: Cas9 protein;
c: an oligodeoxyribonucleic acid or a milt DNA fragment.

23. The kit for use in gene knockout according to claim 22, wherein the oligodeoxyribonucleic acid is a double-stranded DNA with a length of 100-250 bp or a single-stranded DNA with a length of 100-250 nt.

24. The kit for use in gene knockout of a gene according to claim 22, wherein the Cas9 protein is a Cas9 protein derived from *Streptococcus pyogenes,* with amino acid sequence of SEQ ID NO: 18.

25. Use of the T cells with target gene knockout of claim 9 for the preparation of anti-tumor drugs.

26. Use of the T cells with target gene knockout of claim 9 for the preparation of a medicament for preventing or treating infectious diseases caused by virus or bacteria.


Sequence listing

<110>  JIANGSU HENGRUI MEDICINE CO.,LTD., SHANGHAI HENGRUI PHARMACEUTICAL
CO.LTD.

<120>  METHOD FOR KNOCKING OUT TARGET GENE IN T CELL IN VITRO AND crRNA USED
IN THE METHOD

<130>  780046CPCT

<160>  19

<170>  PatentIn version 3.3

<210>  1
<211>  18
<212>  RNA
<213>  artificial sequence

<220>
<223>  crRNA-1

<400>  1
   caaauguguc acaaagua                                                    18


<210>  2
<211>  18
<212>  RNA
<213>  artificial sequence

<220>
<223>  crRNA-2

<400>  2
aaaacugugc uagacaug                                                     18


<210>  3
<211>  18
<212>  RNA
<213>  artificial sequence

<220>
<223>  crRNA-3

<400>  3
ucaagagcaa cagugcug                                                     18


<210>  4
<211>  18
<212>  RNA
<213>  artificial sequence

<220>
<223>  crRNA-4

<400>  4
caccuucuuc cccagccc                                                     18


<210>  5

<211>  18
<212>  RNA
<213>  artificial sequence

<220>
<223>  crRNA-5

<400>  5
gaauaaugcu guuguuga                                                    18


<210>  6
<211>  18
<212>  RNA
<213>  artificial sequence

<220>
<223>  crRNA-6

<400>  6
gauuuagagu cucucagc                                                    18


<210>  7
<211>  18
<212>  RNA
<213>  artificial sequence

<220>
<223>  crRNA-7

<400>  7
acggcagggu caggguuc                                                    18


<210>  8
<211>  18
<212>  RNA
<213>  artificial sequence

<220>
<223>  crRNA-8

<400>  8
guuccuguga ugucaagc                                                    18


<210>  9
<211>  18
<212>  RNA
<213>  artificial sequence

<220>
<223>  crRNA-9

<400>  9
ucaaaaccug ucagugau                                                    18


<210>  10
<211>  18
<212>  RNA
<213>  artificial sequence

<220>
<223>  crRNA-10

<400>  10
gaauccuccu ccugaaag                                                    18


<210>  11
<211>  17
<212>  RNA
<213>  artificial sequence

<220>
<223>  crRNA-11

<400>  11
gguacacggc aggguca                                                     17


<210>  12
<211>  20
<212>  RNA
<213>  artificial sequence

<220>
<223>  crRNA-12

<400>  12
gagaaucaaa aucggugaau                                                  20


<210>  13
<211>  18
<212>  RNA
<213>  artificial sequence

<220>
<223>  crRNA-13

<400>  13
guagcgcgag cacagcua                                                    18


<210>  14
<211>  20
<212>  RNA
<213>  artificial sequence

<220>
<223>  crRNA-14

<400>  14
cgacuggcca gggcgccugu                                                  20


<210>  15
<211>  18
<212>  RNA
<213>  artificial sequence

<220>
<223>  crRNA-15

<400>  15
gugcuacaac ugggcugg                                                    18


<210>  16
<211>  20
<212>  RNA
<213>  artificial sequence

<220>
<223>  crRNA-16

<400>  16
ggcgcccugg ccagucgucu                                                  20


<210>  17
<211>  80
<212>  RNA
<213>  artificial sequence

<220>
<223>  tracrRNA

<400>  17
guuuuagagc uagaaauagc aaguuaaaau aaggcuaguc cguuaucaac uugaaaaagu      60

ggcaccgagu cggugcuuuu                                                  80


<210>  18
<211>  1378
<212>  PRT
<213>  artificial sequence

<220>
<223>  Amino acid sequence of the Cas9(including NLS) protein

<400>  18

Met Asp Lys Lys Tyr Ser Ile Gly Leu Ala Ile Gly Thr Asn Ser Val
1               5                   10                  15


Gly Trp Ala Val Ile Thr Asp Glu Tyr Lys Val Pro Ser Lys Lys Phe
            20                  25                  30


Lys Val Leu Gly Asn Thr Asp Arg His Ser Ile Lys Lys Asn Leu Ile
        35                  40                  45


Gly Ala Leu Leu Phe Asp Ser Gly Glu Thr Ala Glu Ala Thr Arg Leu
    50                  55                  60


Lys Arg Thr Ala Arg Arg Arg Tyr Thr Arg Arg Lys Asn Arg Ile Cys
65                  70                  75                  80


Tyr Leu Gln Glu Ile Phe Ser Asn Glu Met Ala Lys Val Asp Asp Ser
                85                  90                  95

Phe Phe His Arg Leu Glu Glu Ser Phe Leu Val Glu Glu Asp Lys Lys
            100                 105                 110

His Glu Arg His Pro Ile Phe Gly Asn Ile Val Asp Glu Val Ala Tyr
        115                 120                 125

His Glu Lys Tyr Pro Thr Ile Tyr His Leu Arg Lys Lys Leu Val Asp
    130                 135                 140

Ser Thr Asp Lys Ala Asp Leu Arg Leu Ile Tyr Leu Ala Leu Ala His
145                 150                 155                 160

Met Ile Lys Phe Arg Gly His Phe Leu Ile Glu Gly Asp Leu Asn Pro
                165                 170                 175

Asp Asn Ser Asp Val Asp Lys Leu Phe Ile Gln Leu Val Gln Thr Tyr
            180                 185                 190

Asn Gln Leu Phe Glu Glu Asn Pro Ile Asn Ala Ser Gly Val Asp Ala
        195                 200                 205

Lys Ala Ile Leu Ser Ala Arg Leu Ser Lys Ser Arg Arg Leu Glu Asn
    210                 215                 220

Leu Ile Ala Gln Leu Pro Gly Glu Lys Lys Asn Gly Leu Phe Gly Asn
225                 230                 235                 240

Leu Ile Ala Leu Ser Leu Gly Leu Thr Pro Asn Phe Lys Ser Asn Phe
                245                 250                 255

Asp Leu Ala Glu Asp Ala Lys Leu Gln Leu Ser Lys Asp Thr Tyr Asp
            260                 265                 270

Asp Asp Leu Asp Asn Leu Leu Ala Gln Ile Gly Asp Gln Tyr Ala Asp
        275                 280                 285

Leu Phe Leu Ala Ala Lys Asn Leu Ser Asp Ala Ile Leu Leu Ser Asp
    290                 295                 300

Ile Leu Arg Val Asn Thr Glu Ile Thr Lys Ala Pro Leu Ser Ala Ser
305                 310                 315                 320

Met Ile Lys Arg Tyr Asp Glu His His Gln Asp Leu Thr Leu Leu Lys
                325                 330                 335

Ala Leu Val Arg Gln Gln Leu Pro Glu Lys Tyr Lys Glu Ile Phe Phe

            340                 345                 350

Asp Gln Ser Lys Asn Gly Tyr Ala Gly Tyr Ile Asp Gly Gly Ala Ser
        355                 360                 365

Gln Glu Glu Phe Tyr Lys Phe Ile Lys Pro Ile Leu Glu Lys Met Asp
    370                 375                 380

Gly Thr Glu Glu Leu Leu Val Lys Leu Asn Arg Glu Asp Leu Leu Arg
385                 390                 395                 400

Lys Gln Arg Thr Phe Asp Asn Gly Ser Ile Pro His Gln Ile His Leu
                405                 410                 415

Gly Glu Leu His Ala Ile Leu Arg Arg Gln Glu Asp Phe Tyr Pro Phe
            420                 425                 430

Leu Lys Asp Asn Arg Glu Lys Ile Glu Lys Ile Leu Thr Phe Arg Ile
        435                 440                 445

Pro Tyr Tyr Val Gly Pro Leu Ala Arg Gly Asn Ser Arg Phe Ala Trp
    450                 455                 460

Met Thr Arg Lys Ser Glu Glu Thr Ile Thr Pro Trp Asn Phe Glu Glu
465                 470                 475                 480

Val Val Asp Lys Gly Ala Ser Ala Gln Ser Phe Ile Glu Arg Met Thr
                485                 490                 495

Asn Phe Asp Lys Asn Leu Pro Asn Glu Lys Val Leu Pro Lys His Ser
            500                 505                 510

Leu Leu Tyr Glu Tyr Phe Thr Val Tyr Asn Glu Leu Thr Lys Val Lys
        515                 520                 525

Tyr Val Thr Glu Gly Met Arg Lys Pro Ala Phe Leu Ser Gly Glu Gln
    530                 535                 540

Lys Lys Ala Ile Val Asp Leu Leu Phe Lys Thr Asn Arg Lys Val Thr
545                 550                 555                 560

Val Lys Gln Leu Lys Glu Asp Tyr Phe Lys Lys Ile Glu Cys Phe Asp
                565                 570                 575

Ser Val Glu Ile Ser Gly Val Glu Asp Arg Phe Asn Ala Ser Leu Gly
            580                 585                 590

Thr Tyr His Asp Leu Leu Lys Ile Ile Lys Asp Lys Asp Phe Leu Asp
        595                 600                 605

Asn Glu Glu Asn Glu Asp Ile Leu Glu Asp Ile Val Leu Thr Leu Thr
    610                 615                 620

Leu Phe Glu Asp Arg Glu Met Ile Glu Glu Arg Leu Lys Thr Tyr Ala
625                 630                 635                 640

His Leu Phe Asp Asp Lys Val Met Lys Gln Leu Lys Arg Arg Arg Tyr
                645                 650                 655

Thr Gly Trp Gly Arg Leu Ser Arg Lys Leu Ile Asn Gly Ile Arg Asp
            660                 665                 670

Lys Gln Ser Gly Lys Thr Ile Leu Asp Phe Leu Lys Ser Asp Gly Phe
        675                 680                 685

Ala Asn Arg Asn Phe Met Gln Leu Ile His Asp Asp Ser Leu Thr Phe
    690                 695                 700

Lys Glu Asp Ile Gln Lys Ala Gln Val Ser Gly Gln Gly Asp Ser Leu
705                 710                 715                 720

His Glu His Ile Ala Asn Leu Ala Gly Ser Pro Ala Ile Lys Lys Gly
                725                 730                 735

Ile Leu Gln Thr Val Lys Val Val Asp Glu Leu Val Lys Val Met Gly
            740                 745                 750

Arg His Lys Pro Glu Asn Ile Val Ile Glu Met Ala Arg Glu Asn Gln
        755                 760                 765

Thr Thr Gln Lys Gly Gln Lys Asn Ser Arg Glu Arg Met Lys Arg Ile
    770                 775                 780

Glu Glu Gly Ile Lys Glu Leu Gly Ser Gln Ile Leu Lys Glu His Pro
785                 790                 795                 800

Val Glu Asn Thr Gln Leu Gln Asn Glu Lys Leu Tyr Leu Tyr Tyr Leu
                805                 810                 815

Gln Asn Gly Arg Asp Met Tyr Val Asp Gln Glu Leu Asp Ile Asn Arg
            820                 825                 830

Leu Ser Asp Tyr Asp Val Asp His Ile Val Pro Gln Ser Phe Leu Lys
        835                 840                 845

Asp Asp Ser Ile Asp Asn Lys Val Leu Thr Arg Ser Asp Lys Asn Arg
    850                 855                 860

Gly Lys Ser Asp Asn Val Pro Ser Glu Glu Val Val Lys Lys Met Lys
865                 870                 875                 880

Asn Tyr Trp Arg Gln Leu Leu Asn Ala Lys Leu Ile Thr Gln Arg Lys
                885                 890                 895

Phe Asp Asn Leu Thr Lys Ala Glu Arg Gly Gly Leu Ser Glu Leu Asp
            900                 905                 910

Lys Ala Gly Phe Ile Lys Arg Gln Leu Val Glu Thr Arg Gln Ile Thr
        915                 920                 925

Lys His Val Ala Gln Ile Leu Asp Ser Arg Met Asn Thr Lys Tyr Asp
    930                 935                 940

Glu Asn Asp Lys Leu Ile Arg Glu Val Lys Val Ile Thr Leu Lys Ser
945                 950                 955                 960

Lys Leu Val Ser Asp Phe Arg Lys Asp Phe Gln Phe Tyr Lys Val Arg
                965                 970                 975

Glu Ile Asn Asn Tyr His His Ala His Asp Ala Tyr Leu Asn Ala Val
            980                 985                 990

Val Gly Thr Ala Leu Ile Lys Lys  Tyr Pro Lys Leu Glu  Ser Glu Phe
        995                 1000                1005

Val Tyr  Gly Asp Tyr Lys Val  Tyr Asp Val Arg Lys  Met Ile Ala
    1010                1015                1020

Lys Ser  Glu Gln Glu Ile Gly  Lys Ala Thr Ala Lys  Tyr Phe Phe
    1025                1030                1035

Tyr Ser  Asn Ile Met Asn Phe  Phe Lys Thr Glu Ile  Thr Leu Ala
    1040                1045                1050

Asn Gly  Glu Ile Arg Lys Arg  Pro Leu Ile Glu Thr  Asn Gly Glu
    1055                1060                1065

Thr Gly  Glu Ile Val Trp Asp  Lys Gly Arg Asp Phe  Ala Thr Val
    1070                1075                1080

Arg Lys  Val Leu Ser Met Pro  Gln Val Asn Ile Val  Lys Lys Thr
    1085                1090                1095

Glu Val Gln Thr Gly Gly Phe Ser Lys Glu Ser Ile Leu Pro Lys
    1100                1105                1110

Arg Asn Ser Asp Lys Leu Ile Ala Arg Lys Lys Asp Trp Asp Pro
    1115                1120                1125

Lys Lys Tyr Gly Gly Phe Asp Ser Pro Thr Val Ala Tyr Ser Val
    1130                1135                1140

Leu Val Val Ala Lys Val Glu Lys Gly Lys Ser Lys Lys Leu Lys
    1145                1150                1155

Ser Val Lys Glu Leu Leu Gly Ile Thr Ile Met Glu Arg Ser Ser
    1160                1165                1170

Phe Glu Lys Asn Pro Ile Asp Phe Leu Glu Ala Lys Gly Tyr Lys
    1175                1180                1185

Glu Val Lys Lys Asp Leu Ile Ile Lys Leu Pro Lys Tyr Ser Leu
    1190                1195                1200

Phe Glu Leu Glu Asn Gly Arg Lys Arg Met Leu Ala Ser Ala Gly
    1205                1210                1215

Glu Leu Gln Lys Gly Asn Glu Leu Ala Leu Pro Ser Lys Tyr Val
    1220                1225                1230

Asn Phe Leu Tyr Leu Ala Ser His Tyr Glu Lys Leu Lys Gly Ser
    1235                1240                1245

Pro Glu Asp Asn Glu Gln Lys Gln Leu Phe Val Glu Gln His Lys
    1250                1255                1260

His Tyr Leu Asp Glu Ile Ile Glu Gln Ile Ser Glu Phe Ser Lys
    1265                1270                1275

Arg Val Ile Leu Ala Asp Ala Asn Leu Asp Lys Val Leu Ser Ala
    1280                1285                1290

Tyr Asn Lys His Arg Asp Lys Pro Ile Arg Glu Gln Ala Glu Asn
    1295                1300                1305

Ile Ile His Leu Phe Thr Leu Thr Asn Leu Gly Ala Pro Ala Ala
    1310                1315                1320

Phe Lys Tyr Phe Asp Thr Thr Ile Asp Arg Lys Arg Tyr Thr Ser

1325                1330                1335

Thr Lys  Glu Val Leu Asp Ala  Thr Leu Ile His Gln  Ser Ile Thr
    1340                1345                1350

Gly Leu  Tyr Glu Thr Arg Ile  Asp Leu Ser Gln Leu  Gly Gly Asp
    1355                1360                1365

Pro Lys  Lys Lys Arg Lys Val  Met Asp Lys
    1370                1375

<210>  19
<211>  127
<212>  DNA
<213>  artificial sequence

<220>
<223>  random N-oligo

<400>  19
tcatgtggtc ggggtagcgg ctgaagcact gcacgccgta cgtcagggtg gtcacgaggg     60

tgggccaggg cacgggcagc ttgccggtgg tgcagatgaa cttcagggtc agcttgccgt    120

aggtggc                                                              127